(19) Europäisches Patentamt
European Patent Office
Office européen des brevets

(11)  **EP 4 304 541 B1**

(12)  **EUROPEAN PATENT SPECIFICATION**

(45) Date of publication and mention
of the grant of the patent:
**24.06.2026  Bulletin 2026/26**

(21) Application number: **22712196.9**

(22) Date of filing: **08.03.2022**

(51) International Patent Classification (IPC):
***A61F 13/15*** *(2006.01)*      ***A61F 13/513*** *(2006.01)*

(52) Cooperative Patent Classification (CPC):
**A61F 13/15203; A61F 13/51394;** A61F 2013/15243

(86) International application number:
**PCT/US2022/019232**

(87) International publication number:
**WO 2022/192172 (15.09.2022 Gazette 2022/37)**

(54) **ABSORBENT ARTICLE**

ABSORBIERENDER ARTIKEL

ARTICLE ABSORBANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **08.03.2021  US 202163158054 P**

(43) Date of publication of application:
**17.01.2024  Bulletin 2024/03**

(73) Proprietor: **The Procter & Gamble Company Cincinnati, OH 45202 (US)**

(72) Inventors:
• **SARTINI, Emma Lynn**
**Cincinnati, Ohio 45202 (US)**
• **RAMACHANDRAN, Alexandra Joan**
**Cincinnati, Ohio 45202 (US)**

(74) Representative: **P&G Patent Germany**
**Procter & Gamble Service GmbH**
**Sulzbacher Straße 40**
**65824 Schwalbach am Taunus (DE)**

(56) References cited:
**WO-A1-2016/073694      WO-A1-2019/233569**
**US-A1- 2012 310 200**

**Description**

FIELD OF THE INVENTION

The present invention pertains to disposable absorbent articles having a color profile to create visual cues to the user.

BACKGROUND OF THE INVENTION

Disposable absorbent articles such as feminine hygiene pads have been utilized for quite some time. These absorbent articles are utilized to absorb liquid insults, i.e. body exudates. In general, such absorbent articles comprise a topsheet, a backsheet, and an absorbent core disposed between the topsheet and the backsheet.
There are a wide variety of absorbent articles designed to absorb body exudates. These absorbent articles, even when focusing on menstrual pads or adult incontinence pads, come in many shapes and sizes. Their absorbent capacities vary to quite a large extent. So the amount of time that a user can utilize an absorbent article very much depends on the absorbent capacity of the article.
An additional complicating factor is the amount of liquid discharged to the absorbent article. As an example, during menstruation, the amount of discharge is not constant on a day to day basis. So, the amount of time an absorbent article lasted on day one may be quite different from the amount of time the absorbent article lasts on day 4 of the menstruation cycle. The same is true for adult incontinence pads. The amount of time before these pads reach capacity can vary greatly depending on the amount of liquid insult to the pad.
While replacing feminine hygiene pads, including menstrual pads and adult incontinence pads, in a timely manner can help preclude leakage. Placement of the pad within the underwear is just as important. Misaligned pads can cause leakage problems even prior to the pad reaching its full absorbent capacity. This can not only frustrate the user but may also cause quite a bit of embarrassment.
US2012310200A1 relates to absorbent articles which provides a signal viewable from the top surface of the absorbent article. The viewing surface of the absorbent article has a colored portion with at least two shades.
WO20192333569A1 is concerned with arrays comprising at least two disposable pant articles. Each pant has a structure, and at least the outside surface and a surrounding zone of the absorbent core have substantially the same colour. Each of the disposable pants comprises an information tag arranged on the inside of the waist portion, the material of the information tag being in a third colour which is different from the first and second colours by a colour difference ∆E*ab more than 20. The first structure is structurally different from the second structure and/or the first colour is different from the second colour by a colour difference ∆E*ab more than 10.
Based on the foregoing, what is needed is an absorbent article which provides the user with visual cues regarding both capacity and placement. Additionally, it would be beneficial to provide such cues in a simplified manner such that the complexity of manufacturing such articles is not overly complex.

SUMMARY OF THE INVENTION

The invention provides a disposable absorbent article as defined in the claims.

BRIEF DESCRIPTION OF THE DRAWINGS

FIG. 1A is a picture of an absorbent article showing a wearer-facing surface of the absorbent article, wherein the absorbent article is constructed in accordance with the present disclosure.
FIG. 1B is cross section of the absorbent article of FIG. 1A taken along line 1B-1B.
FIG. 1C is a cross section of the absorbent article of FIG. 1A taken along line 1C-1C.
FIG. 2 is a picture of the absorbent article of FIG. 1A showing a garment-facing surface of the absorbent article.
FIG. 3 is a schematic representation of a print portion shown on the garment-facing surface of the absorbent article of FIG. 1A.

DETAILED DESCRIPTION OF THE INVENTION

The term "absorbent article" as used herein refers to devices which absorb and contain exudates, and, more specifically, refers to devices which are placed against or in proximity to the body of the wearer to absorb and contain the various exudates discharged from the body. Absorbent articles of the present disclosure include, but are not limited to, diapers, adult incontinence briefs, training pants, diaper holders, menstrual pads, incontinence pads, liners, absorbent inserts, pantiliners, tampons, and the like.

Absorbent articles of the present disclosure provide the user with placement guides along with highlighted edges of an absorbent core within the absorbent article. The placement guide provides input to the user regarding positioning of the absorbent article on their underwear. Correct placement of the article on the underwear reduces the likelihood of leakage. And, the highlighted edges of the absorbent core can serve as a capacity cue to the user. The capacity cue can provide a signal to the user when the pad is running out of capacity and should be changed. Similar to the placement guides, this feature also reduces the likelihood of leakage.

Absorbent articles of the present disclosure comprise a topsheet, a backsheet, and an absorbent system disposed between the topsheet and the backsheet. The absorbent articles of the present disclosure further comprise a longitudinal axis and a transverse axis extending generally perpendicular to the longitudinal axis. Additionally, absorbent articles of the present disclosure comprise a front portion, a rear portion, and a central portion disposed between the front portion and the rear portion. Each of the front portion, rear portion, and central portion, comprise about one third of an overall length of the absorbent article.

The absorbent article comprises an outer periphery or outer edge. The outer periphery defines the extent of the absorbent article both longitudinally and transversely. The outer periphery typically comprises the outer edge of the absorbent article where the topsheet is joined to the backsheet. The topsheet may be joined to backsheet in any suitable manner, including adhesives, heat sealing, the like, or combinations thereof.

As mentioned previously, the absorbent system is disposed between the topsheet and the backsheet. In addition to comprising the absorbent core, the absorbent system may further comprise additional optional layers. For example, an acquisition / distribution layer may be provided between the topsheet and the backsheet. As another example, a layer which quickly acquires liquid insults from the topsheet may be provided between the topsheet and the absorbent core while a separate primary distribution layer may be provided between the acquisition layer and the absorbent core. As yet another example, a secondary distribution layer may be provided between the absorbent core and the backsheet. This secondary distribution layer may be provided in addition to the acquisition / distribution layer or in addition to the primary acquisition layer and the distribution layer.

In order to provide the capacity and placement cues of the absorbent articles of the present disclosure, one or more colorants is utilized. For example, the capacity cue may comprise a first colorant which is visible from a wearer-facing side of the absorbent article. As another example, the placement cue may comprise a second colorant which is visible from a garment-facing side of the article. The first colorant may be provided on layers of the absorbent article which are more proximal to the topsheet. As an example, in order to highlight the longitudinal side edges and/or the transverse end edges of the absorbent core, the backsheet, one or more layers between the absorbent core and the backsheet, and/or one or more layers between the topsheet and the absorbent core, may comprise a colorant.

The second colorant may be provided on layers of the absorbent article which are more proximal to the backsheet. As an example, the second colorant may be provided on a wearer-facing and/or a garment-facing surface of the backsheet and/or a secondary distribution layer.

While the first colorant and second colorant may be provided as described previously, such applications of colorant can create manufacturing complexities in that there are two separate layers which comprise printing which can mean increased costs due to the amount of colorant being provided. However, forms are contemplated where colorant associated with one or more layers of the absorbent articles may be utilized to accommodate both the capacity cue as well as the placement cue.

In order to further understand this simplified form, some information regarding zones of the absorbent article must be discussed. Absorbent articles of the present disclosure comprise a plurality of zones. One of the primary zones is the absorbent core zone. The absorbent core zone is defined by longitudinal side edges and end edges of the absorbent core. The absorbent core zone includes portions of all layers of the absorbent article which overlap the absorbent core and are within the bounds of the longitudinal side edges and transverse end edges of the absorbent core.

The absorbent articles of the present disclosure similarly comprise an outer boundary zone and an inner boundary zone. The outer boundary zone comprises the outer periphery of the absorbent article and extends inboard thereof. For example, the outer boundary zone can extend to about 30 mm or less inboard of the outer periphery. In one specific example, the outer boundary zone may comprise the periphery of the absorbent article in the rear portion of the absorbent article. Additionally, the outer boundary zone may comprise the periphery, or a portion of the periphery in the central region of the absorbent article.

The outer boundary zone may comprise a plurality of layers of the absorbent article. As an example, the outer boundary zone may comprise the topsheet and the backsheet. The outer boundary zone may comprise the acquisition /distribution layer, primary distribution layer, and/or secondary distribution layer.

The inner boundary zone is disposed between the outer boundary zone and the absorbent core zone. The inner boundary zone comprises a portion of all of the layers of the absorbent article within the inner boundary zone, with the exception of the absorbent core. Much like the outer boundary zone, the inner boundary zone may comprise the topsheet and the backsheet and additionally may comprise the acquisition/distribution layer, primary distribution layer, and/or secondary distribution layer. The inner boundary zone may be provided mainly in the rear portion of the absorbent article. For example, the inner boundary zone may have a larger extent, e.g. larger surface area, in the rear portion of the absorbent article than in the central portion and/or front portion of the absorbent article.

In addition to the layers of the absorbent article mentioned heretofore or independently thereof, another layer may be provided. For example, a support layer may be disposed between the absorbent core and the backsheet or between the topsheet and the backsheet. The support layer may be any suitable material, e.g. nonwoven, film, or combinations thereof. Support layers may be particularly useful in pads which are designed for overnight use.

The support layer may be disposed, in part, in the absorbent core zone, inner boundary zone, and optionally the outer boundary zone. Additionally, the support layer may be disposed in the rear portion and potentially partially in the central portion. In some forms, the support layer may be absent from the front portion. Support layers as well as overnight pads are described in additional detail in U.S. Patent No. 8,715,258 B2, issued to Munakata et al.

With the understanding of the zones of the absorbent article, we now return to the use of colorant to provide the capacity and placement cues. As noted, a colorant associated with one or more layers can provide both the capacity and placement cues. However placement of the colorant is critical to accommodating both cues. For example, colorant which is disposed in an absorbent core zone, e.g. between the absorbent core and the backsheet, i.e. viewable from the garment-facing surface, may be sufficient as a placement cue but may not sufficiently highlight the edges of the absorbent core to provide a capacity cue. Similarly, colorant provided in the absorbent core zone viewable from the wearer-facing surface of the article may sufficiently highlight the edges of the core but may not be visible from the garment-facing surface of the absorbent article.

In general, the absorbent core is opaque and does not allow for colorants disposed on one side of the absorbent core to be viewed on the opposite side. So, for the absorbent article of the present disclosure, colorant can be provided in the inner boundary zone and the outer boundary zone. Colorant provided in the inner boundary zone can provide both the capacity cue and the placement cue. It is also worth noting that more than one colorant may be utilized for the placement and/or capacity cue.

In order for the colorant to be viewed from the wearer-facing surface or the garment-facing surface, the materials within the inner boundary zone and/or outer boundary zone should be selected such that they are not overly opaque and inhibit viewing of the colorant. As an example, several topsheet materials were tested to understand the impact of opacity on the perception of the cues - specifically the capacity cue. Data for the samples tested is provided in Table 1 below.

Table 1

| Sample Description | Basis Weight (grams / meter^2) | Opacity | Perception of Visual Queue |
| --- | --- | --- | --- |
| Sample 1 - NW (non-apertured region) | 24 | 42.6 | pass |
| Sample 1 - NW (apertured region) | 24 | 41.4 | pass |
| Sample 2 - NW | 25 | 27.6 | Pass |
| Sample 3 - NW | 40 | 56.9 | Fail |
| Sample 4 - NW | 40 | 57.1 | Fail |
| Sample 5 - NW | 35 | 51.4 | pass |
| Sample 6 - NW | 24 | 36.8 | Pass |
| Sample 7 - NW | 24 | 41.6 | Pass |
| Sample 8 - NW | 24 | 42.2 | pass |
| Sample 9 - NW | 42 | 58.8 | Fail |

Sample 1 was a carded nonwoven material comprising 100 percent bico fibers having a denier of 2, which were polyethylene (PE) / polyethylene terephthalate (PET) with 1.5 percent titanium dioxide.

Sample 2 was a nonwoven material comprising 100 percent bico fibers which were polypropylene (PP) and PE with 1.5 percent titanium dioxide.

Sample 3 - not according to the claims - was a nonwoven material comprising 1.5 denier bico fibers of PE and PET with 1.5 percent titanium dioxide.

Sample 4 - not according to the claims - was a nonwoven having 2 denier bico fibers of PE and PET with 1.5 percent titanium dioxide.

Samples 5, which is not according to the claims, and 6 were nonwovens having 3 denier spiral bico fibers of PE/PET and 2 denier bico fibers of PE/PET, with 1.5 percent titanium dioxide.

Sample 7 was a nonwoven material having 2.5 denier bico fibers of PE/PET and 2 denier bico fibers of PE/PET, with 1.5 percent titanium dioxide.

Sample 8 was a nonwoven material having 2.5 denier spiral bico fibers of PE/PET and 2 denier bico fibers of PE/PET, with 1.5 percent titanium dioxide.

Sample 9 - not according to the claims - was a nonwoven material comprising 100 percent bico fibers having a denier of 2, which were polyethylene (PE) / polyethylene terephthalate (PET) with 1.5 percent titanium dioxide. While Sample 9 had a similar material construction to that of Sample 1, Sample 9 comprises an aperture pattern which was a floral pattern.

It is worth noting that film topsheets may also be utilized as long as they have a similar opacity as what is demonstrated in Table 1 regarding those materials which achieved a score of "pass." The score of "pass" is a subjective metric which focuses on a materials ability to allow underlying colorant to be viewed by the user. So for those materials which received a score of "fail," it was determined that their opacity was too high to allow for the edges of the absorbent core to be highlighted sufficiently.

While the basis weights shown in Table 1 are nominal (target basis weights), it is believed that the actual basis weights are within plus or minus 5 percent of the target basis weight. Based on the data in Table 1, the inventors believe that the topsheet should have a basis weight of no greater than 35 gsm in order to meet allow sufficient visibility for the capacity cue. The topsheet may have a basis weight of 35 gsm or less, more preferably 30 gsm or less, or most preferably 27 gsm or less, specifically reciting all values within these ranges and any ranges created thereby.

Topsheets of the present disclosure have an opacity of less than 51, or most preferably less than 47, specifically reciting all values within these ranges and any ranges created thereby. And while in theory opacities which are very low would tend to facilitate the visualization of the cues mentioned heretofore, topsheets with very low opacities will also tend to show more vividly the staining caused by the liquid insults. This can be very disturbing to some users, particularly where the liquid insult is menses. So, the topsheets of the present disclosure should have a sufficient level of opacity to allow for the visualization of the cues but also to sufficiently mask staining caused by the liquid insults to the absorbent article. With this in mind, topsheets of the present disclosure can have an opacity of from between about 23 to about 51, or most preferably from about 25 to about 47, specifically reciting all values within these ranges and any ranges created thereby.

It is further believed that the foregoing opacity values may similarly apply to multiple layers of the absorbent article. For example, where a support layer is disposed between the topsheet and the absorbent core and the colorant is disposed on the backsheet, collectively, the support layer and the topsheet can be within the opacity ranges described heretofore. In this same example, where the colorant is disposed on a wearer-facing surface of the backsheet, the backsheet may have similar opacity values discussed herein such that the placement cue is easily visible to the user. In yet another example where the colorant is disposed on the garment-facing surface of the absorbent article, the collective opacity of the backsheet, optional layers between the topsheet and the backsheet, and the topsheet can have the opacity values described herein. In such constructions, as noted previously, it may be beneficial to select the topsheet such that its opacity is higher than that of the optional layers as well as that of the backsheet. Or alternatively, the topsheet and backsheet may be selected such that their opacities are similar while the optional layers between the topsheet and the backsheet have lower opacity than that of either the topsheet or the backsheet. Selection of higher opacity topsheets and/or backsheets can help mask stains within the pad.

With the topsheet and any other intervening layers chosen as described above, the absorbent core zone, inner boundary zone, and outer boundary zone may have distinct visual appearances. These distinct visual appearances set up the cues discussed herein. The differences in visual appearance between the absorbent core zone, inner boundary zone, and outer boundary zone, are described herein within the context of the Hunter color scale. In the Hunter color scale, several values are measured. The lightness or darkness of a sample is measured as L*. Higher values of L* are indicative of lighter colors while lower values are indicative of darker colors. Red versus green of a sample is measured as a*. A positive number indicates red and a negative number indicates green. Yellow versus blue of a sample is measured as b*. A positive number indicates yellow and a negative number indicates blue. The distance between two colors in color space is denoted as delta E* or $\Delta$E*. Within the context of disposable absorbent articles, it is believed that visible differences between samples occurs at as low as a $\Delta$E* of 2. Delta E* is discussed in additional detail in the TEST METHODS section of this specification.

The absorbent core zone will generally comprise a background color, e.g. white. So, the inner boundary zone should comprise a contrasting color, e.g. a difference in a*, b*, L* or delta E*. Similarly, in order to simplify the creation of the cues, the outer boundary zone can have a contrasting color value to that of the inner boundary zone and the absorbent core zone. The absorbent core exhibits a first L* value, the outer boundary zone exhibits a second L* value, and the inner boundary zone exhibits a third L* value. The second L* value is less than about 89. And, the third L* value is greater than the second L* value, and the first L* value can be greater than the second L* value.

As noted, the absorbent core zone typically comprises a background color, e.g. white. So, the first L* value can typically be fairly high. As an example, the first L* value can be greater than about 94 or between 94 to 97. Note while the first L* value may be lower than 94, in such constructions, the third L* value should be lower than the first L* value by at least 4 to ensure that the inner boundary zone is visibly distinct from the absorbent core zone. The difference in L* values between the absorbent core zone and the inner boundary zone can ensure that the edges of the absorbent core are highlighted sufficiently to provide the visual capacity cue as described herein.

The inner boundary zone comprises the third L* value which is lower than the first L* value and greater than the second L* value. The third L* value can be between 60 and 94, more preferably from between about 65 to about 92, or most preferably from about 70 to about 90.

The outer boundary zone comprises the second L* value which is lower than both the first L* value and the second L* value. The second L* value can be between about 50 to about 91, more preferably from about 50 to about 89, or most preferably from about 50 to about 83.

As noted previously, coupled with the difference in L* values, the absorbent core zone, inner boundary zone, and outer boundary zone, may comprise differences in the green - red measurement and yellow - blue measurement as well. As an example, a first delta E* value between the absorbent core zone and the inner boundary zone can be 4 or more, more preferably 7 or more, or most preferably 9 or more, specifically reciting all values within this range and any range created thereby. For example, the first delta E* can be between about 4 to about 18, or more preferably from about 4 to about 15, or most preferably from about 4 to about 13, specifically reciting all values within these ranges and any ranges created thereby.

Similarly a second delta E* between the inner boundary zone and the outer boundary zone can be greater than 4, more preferably greater than 6, or most preferably greater than 8. The first delta E* may be less than the second delta E*. In contrast, in some forms, the first delta E* may be greater than the second delta E*. Exemplary absorbent articles constructed in accordance with the present disclosure were created and compared to an absorbent article not in accordance with the present disclosure. The results are provided in Table 2 below.

Table 2

| Sample No. | L* | a* | b* | C* |
|---|---|---|---|---|
| Sample 1 - wings | 72.37 | 5.81 | -16.47 | 17.5 |
| Sample 1 - inner boundary zone | 87.82 | 1.03 | -4.12 | 4.2 |
| Sample 1 - absorbent core zone | 96.05 | -0.63 | 3.05 | 3.1 |
| Sample 1 - outer boundary zone | 80.23 | 3.24 | -9.65 | 10.2 |
| Sample 2 - wings | 87.05 | 1.90 | -7.49 | 7.7 |
| Sample 2 - inner boundary zone | 92.58 | -0.01 | -1.87 | 1.9 |
| Sample 2 - absorbent core zone | 95.97 | -0.57 | 3.29 | 3.3 |
| Sample 2 - outer boundary zone | 88.78 | 0.84 | -4.39 | 4.5 |
| Sample 3 - wings | 90.5 | 0.5 | -4.01 | 4.0 |
| Sample 3 - inner boundary zone | 94.5 | -0.56 | -0.32 | 0.6 |
| Sample 3 - absorbent core zone | 94.85 | -0.58 | 2.81 | 2.9 |
| Sample 3 - outer boundary zone | 89.77 | 0.53 | -3.45 | 3.5 |

Table 3 below shows the differences in L*, a*, b*, C*, as well as the ΔE* between various locations on the sample articles.

Table 3

| Sample No. and location | ΔL* | Δa* | Δb* | ΔC* | ΔE* |
|---|---|---|---|---|---|
| Sample 1 - wings / inner boundary zone | -15.45 | 4.78 | -12.35 | -13.22 | 20.35 |
| Sample 1- absorbent core zone / inner boundary zone | 8.23 | -1.66 | 7.17 | -1.13 | 11.04 |
| Sample 1 - inner boundary zone / outer boundary zone | 7.59 | -2.21 | 5.53 | -5.94 | 9.65 |
| Sample 1 - absorbent core zone / outer boundary zone | 15.82 | -3.97 | 12.70 | -7.07 | 20.65 |
| Sample 2 - wings / inner boundary zone | -5.53 | 1.91 | -5.62 | -5.86 | 8.11 |
| Sample 2- absorbent core zone / inner boundary zone | 3.39 | -0.51 | 5.16 | 1.46 | 6.19 |
| Sample 2 - inner boundary zone / outer boundary zone | 3.80 | -0.85 | 2.52 | -2.60 | 4.64 |

(continued)

| Sample No. and location | ΔL* | Δa* | Δb* | ΔC* | ΔE* |
|---|---|---|---|---|---|
| Sample 2 - absorbent core zone / outer boundary zone | 7.19 | -0.32 | 7.68 | -1.14 | 10.52 |
| Sample 3 - wings / inner boundary zone | -3.55 | 1.06 | -3.69 | -3.40 | 5.23 |
| Sample 3- absorbent core zone / inner boundary zone | 0.8 | -0.02 | 3.13 | 2.22 | 3.23 |
| Sample 3 - inner boundary zone / outer boundary zone | 4.28 | -1.09 | 3.13 | -2.85 | 5.42 |
| Sample 3 - absorbent core zone / outer boundary zone | 5.08 | -1.11 | 6.26 | -0.63 | 8.14 |

Samples 1 and 2 were constructed in accordance with the present disclosure and Sample 3 was not. Each of Samples 1-3 comprised a 24 gsm topsheet and a film backsheet. Between the film backsheet was a 50 gsm spunlace secondary topsheet and an airlaid absorbent core having a basis weight of 69 gsm. The backsheets for Samples 1 and 2 comprised a higher basis weight of colorant than did the backsheet for Sample 3.

The colorant utilized to create the cues described herein may be provided on any suitable layer or combination of layers of the absorbent article. Additionally, colorant(s) may be utilized to provide other visual cues on the wearer-facing surface and/or the garment-facing surface. As an example, the topsheet, or a layer subjacent to the topsheet may comprise colorant. For example, the topsheet may comprise colorant on an underside thereof. In combination or independently thereof, a subjacent layer to the topsheet may comprise colorant.

The backsheet comprises colorant. This colorant may be disposed on a wearer-facing side of the backsheet and/or a garment-facing surface of the backsheet. In order to provide the placement cue, colorant on the backsheet can be provided in a first portion and a second portion. The first portion of colorant may be disposed on a fist side of the absorbent core zone while the second portion of colorant may be disposed on a second side of the absorbent core zone opposite from the first side. A space which exists between the first portion and the second portion (the absorbent core zone) may be devoid of colorant, may have a lower basis weight of colorant than the first portion and/or second portion, and/or may have a contrasting color from those of the first portion and/or second portion.

The space between the first portion and the second portion extends generally in the longitudinal direction and may extend the full length of the absorbent article or may be shorter than the full length of the absorbent article. As an example, the space may have a length that is at least 50 percent of the overall length of the absorbent article, more preferably at least 75 percent, or most preferably at least 85 percent. In one specific example, the space between the first portion and the second portion may extend the full length of the absorbent article. In order to accommodate registration of the absorbent core and the absorbent core zone, inner edges of the first portion of colorant and/or the second portion of colorant may comprise a scalloped, sinusoidal or any other shape with curved designs of repeating elements. During manufacturing, the placement of the absorbent core, and therefore the absorbent core zone, in relation to the first portion and the second portion, may shift from product to product due to web tracking in a cross-machine direction (transverse direction). The shape of the inner edges of the first portion and second portion can accommodate this shifting of the absorbent core zone. Of import however, is the amplitude (height) of the crests of the curved design as well as the frequency (crests per cm) of the curved design. If the height of the crests is too great, then the colorant disposed in the first portion and/or second portion can appear jagged and uncomfortable. Where the height of the crests is too low, the curved design may not accommodate the variability of the location of the absorbent core zone.

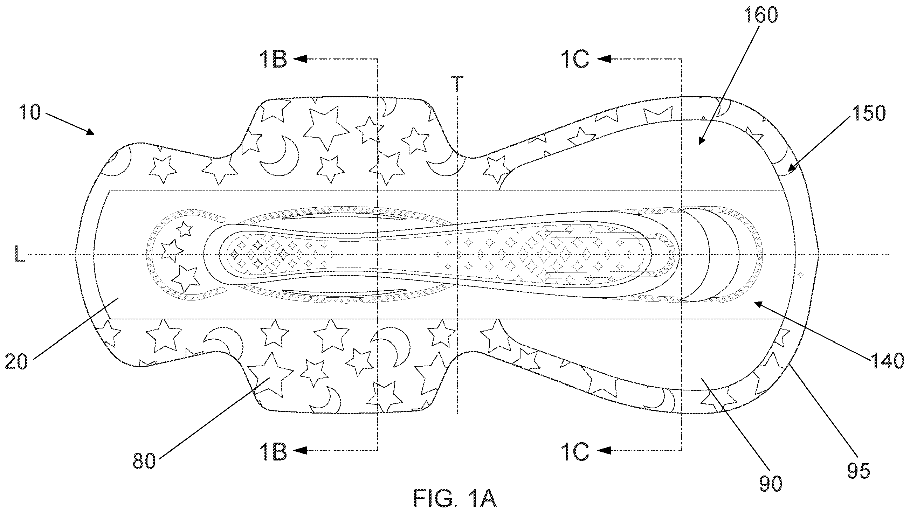

The above is further described in the following figures. As shown in FIGS. 1A-2, an absorbent article 10 comprises a longitudinal centerline "L," a transverse centerline "T," which is generally perpendicular to the longitudinal centerline L. And as noted previously, the absorbent article 10 comprises a front portion 100, a rear portion 110, and a central portion 105 disposed between the front portion 100 and the rear portion 110.

The absorbent article 10 further comprises a topsheet 20, an optional layer 30, an absorbent core 40, and a backsheet 50. Recall that the optional layer 30 may comprise an acquisition / distribution layer, separate acquisition and primary distribution layers, and/or a support layer. Additionally optional layer 30, although depicted between the topsheet 20 and the absorbent core 40, may be disposed between the backsheet 50 and the absorbent core 40. In such forms, the optional layer may comprise a secondary distribution layer and/or a support layer. Forms are also contemplated where optional layers are provided between the topsheet 20 and the absorbent core 40 as well as between the absorbent core 40 and the backsheet 50.

Additionally, the absorbent article 10 may comprise a primary pair of wings 80 on opposing sides of the absorbent article. As shown, the topsheet 20 and the backsheet 50 may extend transversely outward and form at least a portion of the primary wings 80. However, the primary wings 80 may be formed from discrete material as well. As an example, a discrete nonwoven material may be joined to the topsheet 20 and/or the backsheet 50 or may be attached somewhere between the topsheet 20 and the backsheet 50 and extend outward therefrom to form the primary wings 80. Forms are also contemplated where the discrete material along with the topsheet 20 and/or backsheet 50 are joined together to form the primary pair of wings 80.

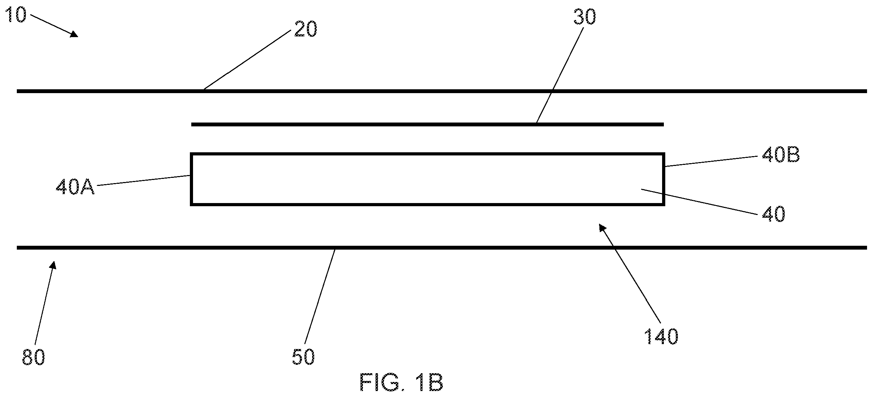

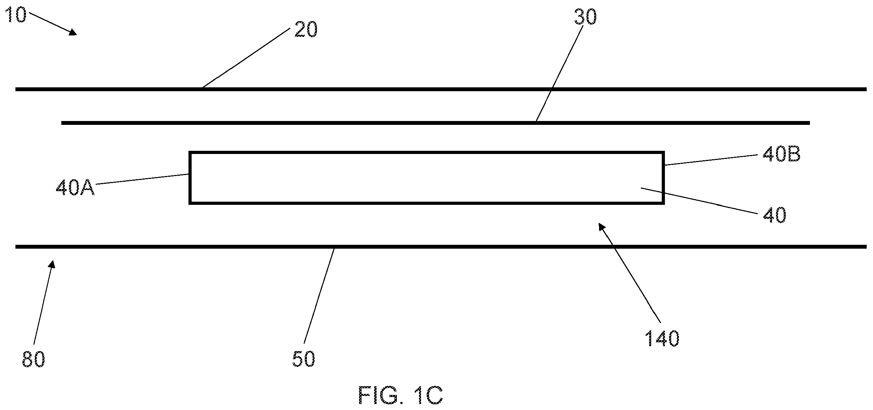

As shown specifically in FIG. 1B, the optional layer 30 may be transversely co-extensive with the absorbent core 40. This particularly may be the case in the front portion 100 and at least part of the central portion 105. However, as shown in FIG. 1C, the optional layer 30 may extend transversely outboard of longitudinal side edges 40A and 40B of the absorbent core 40, particularly in the rear portion 110. The portion of the optional layer 30 which extends transversely beyond the longitudinal side edges 40A and 40B, is disposed in the inner boundary zone 160. As a reminder, the optional layer 30 is one of several options which can be utilized. The other options provided may have a construction similar to that shown in FIG. 1C regarding the transverse extent of the optional layer 30 and may be configured as described herein.

Based on the configuration shown in FIGS. 1B and 1C, an inner boundary zone 160 may be disposed primarily in the rear portion 110 and slightly in the central portion 105. And as shown, the front region 100 may not comprise an inner boundary zone 160. This type of configuration may be beneficial particularly in absorbent articles which are meant for overnight wear. As an example, overnight pads typically have a much longer length than their daily use counterparts. During the evenings, particularly when used during slumber, liquid insults to the pad tend to flow toward the rear portion of the article. So, the longer length of the overnight pads is believed to provide additional capacity to address these liquid insults. This makes the capacity cue (highlighting the edges of the absorbent core) in the rear portion extremely useful.

Referring again to FIGS. 1A-2, an outer periphery 95 is disposed where the topsheet 20 and the backsheet 50 are joined to one another. An outer boundary zone 150 comprises the outer periphery 95 and extends inboard toward the absorbent article. An absorbent core zone 140 is defined by the absorbent core 40 and is bounded by the longitudinal side edges 40A and 40B as well as end edges of the absorbent core 40.

Disposed between the outer boundary zone 150 and the absorbent core zone 140 is the inner boundary zone 160. As shown, the inner boundary zone 160 may form a portion of a secondary pair of wings 90 which extend transversely outward from the absorbent core zone 140. It is worth noting that the secondary pair of wings 90 is typical of overnight pads. So, for absorbent articles which are not overnight pads, the secondary pair of wings 90 is optional.

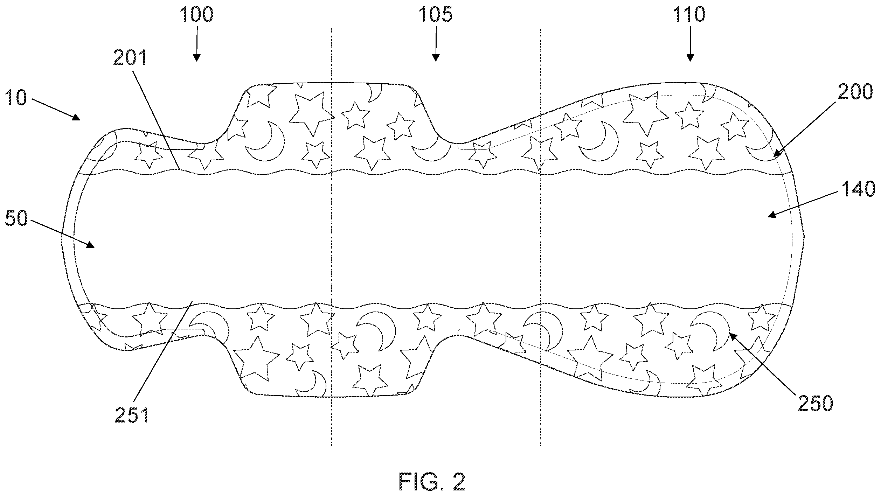

Referring now to FIG. 2, in order to accommodate the cues mentioned herein, the backsheet 50 may comprise colorant. As shown, the backsheet 50 may comprise a first colorant portion 200 and a second colorant portion 250. The first colorant portion 200 and the second colorant portion 250 may be disposed on opposite sides of the absorbent core zone 140.

Each of the first colorant portion 200 and second colorant portion 250 extend transversely outward from the absorbent core zone 140. Additionally, each of the first colorant portion 200 and the second colorant portion 250 comprise an inner edge 201 and 251, respectively. As discussed previously, the inner edges comprise a wave shape. The wave shape of the inner edges 201 and 251 can accommodate the variability of the location of the absorbent core zone 140.

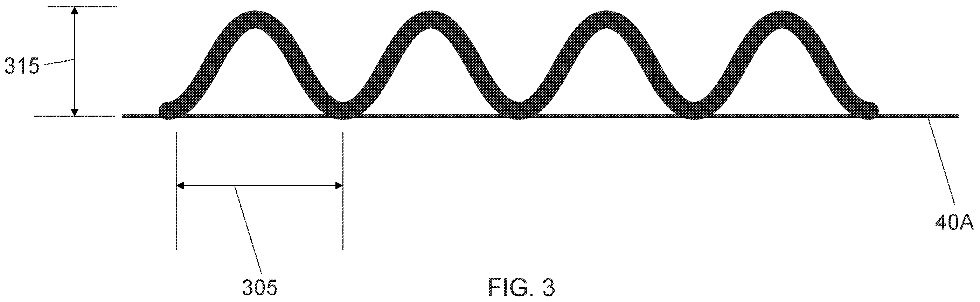

Regarding the curved design of the first portion and/or second portion, we refer to FIG. 3. In one specific example, the curved design may comprise a width 305 of a base that is 2 times or more than a height 315 of a crest. Additionally, an edge of the curved design may cover the variation of the absorbent core placement, when the curved design is off set from the longitudinal side edges of the absorbent core, e.g. 40A, by an amount equal the total placement variation minus 25 percent of the width of the base.

For the specific components of the absorbent article, any suitable materials may be utilized so long as they are in accordance with the requirements disclosed herein. For example, any suitable topsheet may be utilized so long as the opacity of the selected material meets the requirements described herein. Additionally, the topsheet may be compliant, soft feeling, and non-irritating to the wearer's skin. Suitable topsheet materials include a liquid pervious material that is oriented towards and contacts the body of the wearer permitting bodily discharges to rapidly penetrate through it without allowing fluid to flow back through the topsheet to the skin of the wearer. The topsheet, while being capable of allowing rapid transfer of fluid through it, may also provide for the transfer or migration of the lotion composition onto an external or internal portion of a wearer's skin.

A suitable topsheet can be made of various materials such as woven and nonwoven materials; apertured film materials including apertured formed thermoplastic films, apertured plastic films, and fiber-entangled apertured films; hydro-formed thermoplastic films; porous foams; reticulated foams; reticulated thermoplastic films; thermoplastic scrims; or combinations thereof.

Apertured film materials suitable for use as the topsheet include those apertured plastic films that are non-absorbent and pervious to body exudates and provide for minimal or no flow back of fluids through the topsheet. Nonlimiting examples of other suitable formed films, including apertured and non-apertured formed films, are more fully described in U.S. Patent No. 3,929,135, issued to Thompson on December 30, 1975; U.S. Patent No. 4,324,246, issued to Mullane et al. on April 13, 1982; U.S. Patent No. 4,342,314, issued to Radel et al. on August 3, 1982; U.S. Patent No. 4,463,045, issued to Ahr et al. on July 31, 1984; U.S. Patent No. 5,006,394, issued to Baird on April 9, 1991; U.S. Patent No. 4,609,518, issued to Curro et al. on September 2, 1986; and U.S. Patent No. 4,629,643, issued to Curro et al. on December 16, 1986.

Nonlimiting examples of woven and nonwoven materials suitable for use as the topsheet include fibrous materials made from natural fibers, e.g. cotton, including 100 percent organic cotton, modified natural fibers, synthetic fibers, or combinations thereof. These fibrous materials can be either hydrophilic or hydrophobic, but it is preferable that the topsheet be hydrophobic or rendered hydrophobic. As an option, portions of the topsheet can be rendered hydrophilic, using any known method for making topsheets containing hydrophilic components. Nonwoven fibrous topsheets 20 may be produced by any known procedure for making nonwoven webs, nonlimiting examples of which include spunbonding, carding, wet-laid, air-laid, meltblown, needle-punching, mechanical entangling, thermo-mechanical entangling, and hydroentangling.

The topsheet may be formed from a combination of an apertured film and a nonwoven. For example, a film web and a nonwoven web can be combined as described in U.S. Patent No. 9,700,463. Alternatively, a film may be extruded onto a nonwoven material which is believed to provide enhanced contact between the film layer and the nonwoven material. Exemplary processes for such a combination are described in U.S. Patent Nos. 9,849,602 and 9,700,463.

Additionally, the basis weight of these materials can be less than 40 gsm, more preferably less than 35 gsm, or most preferably less than 30 gsm. As another example, the basis weight of the topsheet material can be between about 15 gsm to about 40 gsm, more preferably from about 15 gsm to about 35 gsm, or most preferably from about 15 gsm to about 30 gsm, specifically including any values within these ranges and any ranges created thereby.

The backsheet may be positioned adjacent a garment-facing surface of the absorbent core and may be joined thereto by attachment methods such as those well known in the art. For example, the backsheet may be secured to the absorbent core and/or any layers therebetween by a uniform continuous layer of adhesive, a patterned layer of adhesive, or an array of separate lines, spirals, or spots of adhesive. Alternatively, the attachment methods may comprise using heat bonds, pressure bonds, ultrasonic bonds, dynamic mechanical bonds, or any other suitable attachment methods or combinations of these attachment methods as are known in the art.

The backsheet may be impervious, or substantially impervious, to liquids (e.g., urine) and may be manufactured from a thin plastic film, although other flexible liquid impervious materials may also be used. As used herein, the term "flexible" refers to materials which are compliant and will readily conform to the general shape and contours of the human body. The backsheet may prevent, or at least inhibit, the exudates absorbed and contained in the absorbent core from wetting articles of clothing which contact the incontinence pad such as undergarments. However, the backsheet may permit vapors to escape from the absorbent core (i.e., is breathable) while in some cases the backsheet may not permit vapors to escape (i.e., non-breathable). Thus, the backsheet may comprise a polymeric film such as thermoplastic films of polyethylene or polypropylene. A suitable material for the backsheet is a thermoplastic film having a thickness of from about 0.012 mm (0.5 mil) to about 0.051 mm (2.0 mils), for example. Any suitable backsheet known in the art may be utilized with the present invention.

The backsheet acts as a barrier to any absorbed bodily fluids that may pass through the absorbent core to the garment surface thereof with a resulting reduction in risk of staining undergarments or other clothing. A preferred material is a soft, smooth, compliant, liquid and vapor pervious material that provides for softness and conformability for comfort, and is low noise producing so that movement does not cause unwanted sound.

Exemplary backsheets are described in US Patent Nos. 5,885,265 (Osborn, III.) issued March 23, 1999; 6,462,251 (Cimini) issued October 8, 2002; 6,623,464 (Bewick-Sonntag) issued September 23, 2003 or US Patent No. 6,664439 (Arndt) issued December 16, 2003. Suitable dual or multi-layer breathable backsheets for use herein include those exemplified in U.S. Pat. No. 3,881,489, U.S. Pat. No. 4,341,216, U.S. Pat. No. 4,713,068, U.S. Pat. No. 4,818,600, EP 203 821, EP 710 471, EP 710 472, and EP 793 952.

Suitable breathable backsheets for use herein include all breathable backsheets known in the art. In principle there are two types of breathable backsheets, single layer breathable backsheets which are breathable and impervious to liquids and backsheets having at least two layers, which in combination provide both breathability and liquid imperviousness. Suitable single layer breathable backsheets for use herein include those described for example in GB A 2184 389, GB A 2184 390, GB A 2184 391, U.S. Pat. No. 4,591,523, U.S. Pat. No. 3 989 867, U.S. Pat. No. 3,156,242 and WO 97/24097.

The backsheet may be a nonwoven web having a basis weight between about 20 gsm and about 50 gsm. As an example, the backsheet can be a relatively hydrophobic 23 gsm spunbonded nonwoven web of 4 denier polypropylene fibers available from Fiberweb Neuberger, under the designation F102301001. The backsheet may be coated with a non-soluble, liquid swellable material as described in US Patent No. 6,436,508 (Ciammaichella) issued August 20, 2002.

The backsheet has a garment-facing side and an opposite body-facing side. The garment-facing side of the backsheet comprises a non-adhesive area and an adhesive area. The adhesive area may be provided by any conventional means. Pressure sensitive adhesives have been commonly found to work well for this purpose.

The topsheet may be joined to the backsheet by attachment methods (not shown) such as those well known in the art. The topsheet and the backsheet may be joined directly to each other in the article periphery and may be indirectly joined together by directly joining them to the absorbent core, the fluid management layer, and/or additional layers disposed between the topsheet and the backsheet. This indirect or direct joining may be accomplished by attachment methods which are well known in the art.

The absorbent core may comprise any suitable shape including but not limited to an oval, a discorectangle, a rectangle, an asymmetric shape, and an hourglass. For example, in some forms of the present invention, the absorbent core may comprise a contoured shape, e.g. narrower in the intermediate region than in the end regions. As yet another example, the absorbent core may comprise a tapered shape having a wider portion in one end region of the pad which tapers to a narrower end region in the other end region of the pad. The absorbent core may comprise varying stiffness in the MD and CD.

The configuration and construction of the absorbent core may vary (e.g., the absorbent core may have varying caliper zones, a hydrophilic gradient, a superabsorbent gradient, or lower average density and lower average basis weight acquisition zones). Further, the size and absorbent capacity of the absorbent core may also be varied to accommodate a variety of wearers. However, the total absorbent capacity of the absorbent core should be compatible with the design loading and the intended use of the disposable absorbent article or incontinence pad.

In some forms of the present disclosure, the absorbent core may comprise a plurality of multi-functional layers that are in addition to the first and second laminates. For example, the absorbent core may comprise a core wrap (not shown) useful for enveloping the first and second laminates and other optional layers. The core wrap may be formed by two nonwoven materials, substrates, laminates, films, or other materials. In a form, the core wrap may only comprise a single material, substrate, laminate, or other material wrapped at least partially around itself. The absorbent core may comprise one or more adhesives, for example, to help immobilize the SAP or other absorbent materials within the first and second laminates.

Absorbent cores comprising relatively high amounts of SAP with various core designs are disclosed in U.S. Pat. No. 5,599,335 to Goldman et al., EP 1,447,066 to Busam et al., WO 95/11652 to Tanzer et al., U.S. Pat. Publ. No. 2008/0312622A1 to Hundorf et al., and WO 2012/052172 to Van Malderen. These may be used to configure the superabsorbent layers.

Additions to the core of the present disclosure are envisioned. In particular, potential additions to the current multi-laminate absorbent core are described in U.S. Pat. No. 4,610,678, entitled "High-Density Absorbent Structures" issued to Weisman et al., on Sep. 9, 1986; U.S. Pat. No. 4,673,402, entitled "Absorbent Articles With Dual-Layered Cores", issued to Weisman et al., on Jun. 16, 1987; U.S. Pat. No. 4,888,231, entitled "Absorbent Core Having A Dusting Layer", issued to Angstadt on Dec. 19, 1989; and U.S. Pat. No. 4,834,735, entitled "High Density Absorbent Members Having Lower Density and Lower Basis Weight Acquisition Zones", issued to Alemany et al., on May 30, 1989. The absorbent core may further comprise additional layers that mimic the dual core system containing an acquisition/distribution core of chemically stiffened fibers positioned over an absorbent storage core as detailed in U.S. Pat. No. 5,234,423, entitled "Absorbent Article With Elastic Waist Feature and Enhanced Absorbency" issued to Alemany et al., on Aug. 10, 1993; and in U.S. Pat. No. 5,147,345. These are useful to the extent they do not negate or conflict with the effects of the below described laminates of the absorbent core of the present invention. Additional examples of suitable absorbent cores are described in U.S. Patent Application Publication Nos. 2018/0098893 and 2018/0098891.

Regarding the optional layers of absorbent system, any suitable material may be utilized. Each of these layers may have a basis weight from about 40 gsm to about 100 gsm, from about 45 gsm to about 75 gsm, or from about 50 gsm to about 65 gsm, specifically including all values within these ranges and any ranges created thereby. In some forms, the one or more of these layers may comprise a homogeneous mix of fibers whereas in other forms, the fluid management layer may comprise a heterogeneous mix of fibers.

Some exemplary optional layers for the absorbent system are described in U.S. Patent Application Publication Nos. 2015/0351976 A1 and 2014/0343523 A1; and U.S. Patent Application Serial No. 15/729704.

## TEST METHODS

Color Measurement Method

Color analyses of various zones on an absorbent article are made using a 0°/45° spectrophotometer with adjustable apertures capable of making standard CIE L*a*b* measurements in accordance with ASTM E1349. An example of a suitable spectrophotometer is the Labscan XE (available from Hunter Associates Laboratory, Inc., Reston, VA, or equivalent). All testing is performed in a room maintained at a temperature of 23° C $\pm$ 2.0° C and a relative humidity of 50% $\pm$ 2% and test samples are conditioned under the same environmental conditions for at least 2 hours prior to testing.

For this procedure, the test sample is the intact absorbent article. A total of three individual test samples are required. Determine and note which edge of the sample will be oriented towards the front of the body when in use. Also note which side of the sample is intended to face the body in use. The wearer-facing side of the sample will face the aperture during the measurements. The sample is removed from any outer packaging, unfolded (if necessary), and any release paper that is present is peeled away and disposed. A light coating of talc is applied to the panty fastening adhesive and wing adhesive to mitigate tackiness. The sample is then divided into longitudinal thirds and marked to denote a front portion, a central portion and a rear portion, as follows. The sample is laid flat on a bench and the length of the sample is measured along the longitudinal axis at the transverse midpoint. To mark the front portion, a line that is parallel to the transverse axis is marked on the sample at a distance that is 1/3 the length of the sample away from the front edge. To mark and divide the central and rear portions, a line that is parallel to the transverse axis is marked on the sample at a distance that is 2/3 the length of the sample away from the front edge. The central portion is the section between the lines depicting 1/3 and 2/3 the length of the sample, and the rear portion is the section behind the line depicting 2/3 the length of the sample. In like fashion, draw lines to denote the front, central and rear portions on each of the three samples.

Prior to making any color measurements, the spectrophotometer is calibrated and standardized per the vendor instructions using the standard white and black tiles provided by the vendor. The spectrophotometer must be standardized for each different size aperture just prior to making measurements with each respective aperture. Set the spectrophotometer to use the CIE L*a*b* color space with a D65 standard illumination, a 10° observer, and the UV filter set to nominal. The appropriate size aperture is selected according to which sample zone is being measured, further described herein. The test location for each sample zone is placed flat and centered over the aperture such that the entire aperture is covered by the test location. The standard white tile is held in place on the back side of the test location and a reading is taken. The L*, a*, and b* values are recorded to the nearest 0.01 units.

Referring to FIG. 1A, there are three different zones on the test sample that will undergo color measurements. Zone 1 is the Absorbent Core Zone 140, Zone 2 is the Outer Boundary Zone 150 and Zone 3 is the Inner Boundary Zone 160.

For Zone 1 (absorbent core zone 140), color measurements can be obtained from the front, central or rear portions of the sample, however the test location must be free from added color (e.g print signal or other region of color) with wrinkles and folds avoided. For Zone 1, the aperture size is 0.7 inch in diameter with a 0.50 inch diameter area view. Color readings are obtained from Zone 1 on each of the three test samples and recorded to the nearest 0.01 units. Calculate the arithmetic mean for the L*, a* and b* values obtained across all replicates and report as $L^*_1$, $a^*_1$ and $b^*_1$ to the nearest 0.01 units.

For Zone 3 (inner boundary zone 160), color is measured at a test location that is also free of wrinkles or folds. Zone 3 will most likely reside in the rear portion of the sample. For Zone 3, the aperture size is 0.70 inch in diameter with a 0.50 inch diameter area view. Color readings are obtained from Zone 3 on each of the three test samples and recorded to the nearest 0.01 units. Calculate the arithmetic mean for the L*, a* and b* values obtained across all replicates and report as $L^*_3$, $a^*_3$ and $b^*_3$ to the nearest 0.01 units.

For Zone 2 (outer boundary zone 150), color measurements are recorded to the nearest 0.01 units at multiple test locations in the rear portion of the sample. Due to the small size area in Zone 2, the aperture size is 0.40 inch in diameter with a 0.25 inch diameter area view. The first color reading for Zone 2 is obtained at the first 10 mm location just behind the line that depicts 2/3 the sample length in the rear portion of the sample starting at one of the lateral sides. Subsequent color readings are then taken every 10 mm as the aperture is walked along the entire rear portion of the sample in Zone 2 until the line that depicts 2/3 the sample length is reached on the opposite lateral side of the sample. In like fashion, color readings are obtained from Zone 2 on each of the three test samples and recorded to the nearest 0.01 units. Calculate the arithmetic mean for the L*, a* and b* values obtained across all replicates and report as $L^*_2$, $a^*_2$ and $b^*_2$ to the nearest 0.01 units.

The color difference between the color measurements obtained for the different Zones is calculated as Delta E*. To determine the color difference between Zone 1 (absorbent core zone 140) and Zone 3 (inner boundary zone 160), calculate Delta E* as follows and report to the nearest 0.01 unit, noting the two zones that are being compared:

$$\text{Delta } E^* = \text{Sqrt} \left[ (L_3^* - L_1^*)^2 + (a_3^* - a_1^*)^2 + (b_3^* - b_1^*)^2 \right]$$

To determine the color difference between Zone 2 (outer boundary 150) and Zone 3 (inner boundary 160), calculate Delta E* as follows and report to the nearest 0.01 unit, noting the two zones that are being compared:

$$\text{Delta } E^* = \text{Sqrt} \left[ (L_3^* - L_2^*)^2 + (a_3^* - a_2^*)^2 + (b_3^* - b_2^*)^2 \right]$$

Opacity Measurement Method

Opacity measurements are made using a 0°/45° spectrophotometer with adjustable apertures capable of making standard CIE color measurements using XYZ coordinates and contrast ratio. An example of a suitable spectrophotometer is the Labscan XE (available from Hunter Associates Laboratory, Inc., Reston, VA, or equivalent). Measurements are conducted on either a single layer or multiple layers of test materials. All testing is performed in a room maintained at a temperature of 23° C $\pm$ 2.0° C and a relative humidity of 50% $\pm$ 2% and samples are conditioned under the same environmental conditions for at least 2 hours prior to testing.

Obtain a test sample by removing it from an absorbent article, if necessary. When excising the sample from an absorbent article, use care to not impart any contamination or distortion to the sample layer during the process. The test sample is obtained from an area free of folds or wrinkles, and it must be larger than the aperture being used on the spectrophotometer. Note which side of the test sample is facing (or is meant to face) the body during use. If more than one type of material is to be measured as multiple layers, note the side of each layer that is facing (or is meant to face) the body during use. The wearer-facing surface of the sample(s) is the side that will face the aperture during the measurement. Obtain a sufficient quantity of the sample material(s) such that ten measurements can be made on non-overlapping areas of the material(s) being evaluated.

To measure Opacity, calibrate and standardize the instrument per the vendor instructions using the standard white and black tiles provided by the vendor with a 0.70 inch diameter aperture in place. Set the spectrophotometer to use the CIE XYZ color space with a D65 standard illumination, a 10° observer, 0.70 inch aperture, 0.50 inch area view, and set the UV filter to nominal. Place the body-facing side of the test sample over the aperture and ensure that the entire aperture opening is covered by the sample. If measuring multiple layers, stack them on top of the body-facing layer according to their placement in the absorbent article, centered over the aperture, with the body-facing side of each oriented towards the aperture. Place the standard white tile directly against the back side of the sample(s), take a reading and record the Y value as $Y_{white\ backing}$ to the nearest 0.01 units. Without moving the position of the test sample(s), remove the standard white tile and replace it with the black standard tile. Take a reading and record the Y value as $Y_{black\ backing}$ to the nearest 0.01 units. Calculate Opacity by dividing the $Y_{black\ backing}$ value by the $Y_{white\ backing}$ value and then multiply by 100. Record Opacity to the nearest 0.1 percent, noting the material (or materials) that were measured.

In like fashion, repeat for a total of five measurements on non-overlapping areas of the test sample material(s). Calculate the arithmetic mean for Opacity obtained across all and report to the nearest 0.1 percent, noting the material (or materials) that were measured.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention defined by the claims.

**Claims**

1. A disposable absorbent article (10) having a longitudinal axis and a transverse axis disposed perpendicularly to the longitudinal axis, a front portion (100), a rear portion (110), and a central portion (105) disposed between the front portion (100) and rear portion (110), the disposable absorbent article (10) further comprising:

   a topsheet (20);
   a backsheet (50);
   an absorbent system disposed between the topsheet (20) and the backsheet (50), wherein the absorbent system comprises an absorbent core (40) having longitudinal side edges (40A, 40B) on opposite sides of the absorbent core (40) and end edges on opposite sides of the absorbent core (40), the absorbent core (40) being disposed in an absorbent core zone (140), wherein the absorbent core zone (140) exhibits a first L* value;
   an outer periphery (95) formed at least in part by a portion of the topsheet (20) and a portion of the backsheet (50);
   an outer boundary zone (150) comprising the outer periphery (95), the outer boundary zone (150) having a width of less than 30 mm, wherein the outer boundary zone (150) exhibits a second L* value of less than 89 according to the Color Measurement Method defined herein;
   an inner boundary zone (160) disposed between the outer boundary zone and the absorbent core zone (140) in the rear portion (110), the inner boundary zone (160) exhibits a third L* value according to the Color Measurement Method defined herein which is greater than the second L* value; and
   wherein the first L* value is greater than the second and third L* values such that there is a perceptible visual difference between the absorbent core zone (140) and the inner boundary zone (160);
   wherein the topsheet (20) has an opacity of from 23 to 51 measured according to the opacity measurement method defined herein; and
   wherein colorant is disposed on a surface of the backsheet (50); wherein the L* values are measured according to the Color Measurement method defined herein.

2. The absorbent article of claim 1, wherein the second L* value is from between about 50 and 89 according to the Color Measurement Method defined herein.

3. The absorbent article of either of the preceding claims, wherein the third L* value is from between about 60 and 94 according to the Color Measurement Method defined herein.

4. The absorbent article of any of the preceding claims, wherein a first delta E* value between the absorbent core zone (140) and the inner boundary zone (160) is greater than 4, more preferably greater than 7, or most preferably greater than 9 according to the Color Measurement Method defined herein.

5. The absorbent article of any of the preceding claims, wherein a second delta E* value between the inner boundary zone (160) and the outer boundary zone (150) is greater than 4, more preferably greater than 6, or most preferably greater than 8 according to the Color Measurement Method defined herein.

6. The absorbent article of claim 5, wherein the first delta E* is less than the second delta E* value according to the Color Measurement Method defined herein.

7. The absorbent article of any of the preceding claims, wherein colorant is disposed on a surface of the topsheet (20).

8. The absorbent article of any of the preceding claims, wherein the topsheet (20) comprises a nonwoven material.

9. The absorbent article of any of the preceding claims, wherein the backsheet (50) comprises a colorant, and wherein the colorant is disposed on opposing sides of the absorbent core zone (140).

10. The absorbent article of claim 9, wherein a first colorant portion (200) is disposed on a first side of the absorbent core zone (140), and a second colorant portion (250) is disposed on a second side of the absorbent core zone (140).

11. The absorbent article of any of the preceding claims, further comprising an optional layer (30) disposed between the topsheet (20) and the absorbent core (40) or between the absorbent core (40) and the backsheet (50), wherein the optional layer (30) is disposed in the absorbent core zone (140) and in the inner boundary zone (160), but not the outer boundary zone (150).

12. The absorbent article of claim 11, wherein the optional layer (30) comprises an acquisition / distribution layer or an acquisition layer and a separate distribution layer.

13. The absorbent article of claim 11, wherein the optional layer (30) comprises a support layer which is disposed in the rear portion (110) and central portion (105) but not in the front portion (100).

**Patentansprüche**

1. Einweg-Absorptionsartikel (101), der eine Längsachse und eine Querachse, die lotrecht zu der Längsachse angeordnet ist, einen vorderen Abschnitt (100), einen hinteren Abschnitt (110) und einen mittleren Abschnitt (105), der zwischen dem vorderen Abschnitt (100) und dem hinteren Abschnitt (110) angeordnet ist, aufweist, der Einweg-Absorptionsartikel (10) ferner umfassend:

eine Oberschicht (20);
eine Unterschicht (50);
ein Absorptionssystem, das zwischen der Oberschicht (20) und der Unterschicht (50) angeordnet ist, wobei das Absorptionssystem einen Absorptionskern (40) umfasst, der Längsseitenränder (40A, 40B) auf gegenüberliegenden Seiten des Absorptionskerns (40) und Endränder auf gegenüberliegenden Seiten des Absorptionskerns (40) aufweist, wobei der Absorptionskern (40) in einer Absorptionskernzone (140) angeordnet ist, wobei die Absorptionskernzone (140) einen ersten L*-Wert vorweist;
einen äußeren Umfang (95), der wenigstens teilweise durch einen Abschnitt der Oberschicht (20) und einen Abschnitt der Unterschicht (50) ausgebildet ist;
eine äußere Grenzzone (150), umfassend den äußere Umfang (95), wobei die äußere Grenzzone (150) eine Breite von weniger als 30 mm aufweist, wobei die äußere Grenzzone (150) einen zweiten L*-Wert von weniger als 89 gemäß dem hierin definierten Farbmessverfahren vorweist;
eine innere Grenzzone (160), die zwischen der äußeren Grenzzone und der Absorptionskernzone (140) in dem hinteren Abschnitt (110) angeordnet ist, wobei die innere Grenzzone (160) einen dritten L*-Wert gemäß dem hierin definierten Farbmessverfahren vorweist, der größer als der zweite L*-Wert ist; und

wobei der erste L*-Wert größer als der zweite und dritte L*-Wert ist, derart, dass ein wahrnehmbarer visueller Unterschied zwischen der Absorptionskernzone (140) und der inneren Grenzzone (160) besteht;

wobei die Oberschicht (20) eine Trübung von 23 bis 51 aufweist, gemessen gemäß dem hierin definierten Trübungsmessverfahren; und

wobei Farbstoff auf einer Oberfläche der Unterschicht (50) angeordnet ist; wobei die L*-Werte gemäß dem hierin definierten Farbmessverfahren gemessen werden.

2. Absorptionsartikel nach Anspruch 1, wobei der zweite L*-Wert zwischen etwa 50 und 89 gemäß dem hierin definierten Farbmessverfahren liegt.

3. Absorptionsartikel nach einem der vorstehenden Ansprüche, wobei der dritte L*-Wert zwischen etwa 60 und 94 gemäß dem hierin definierten Farbmessverfahren liegt.

4. Absorptionsartikel nach einem der vorstehenden Ansprüche, wobei ein erster Delta-E*-Wert zwischen der Absorptionskernzone (140) und der inneren Grenzzone (160) größer als 4, mehr bevorzugt größer als 7 oder am meisten bevorzugt größer als 9 gemäß dem hierin definierten Farbmessverfahren ist.

5. Absorptionsartikel nach einem der vorstehenden Ansprüche, wobei ein zweiter Delta-E*-Wert zwischen der inneren Grenzzone (160) und der äußeren Grenzzone (150) größer als 4, mehr bevorzugt größer als 6 oder am meisten bevorzugt größer als 8 gemäß dem hierin definierten Farbmessverfahren ist.

6. Absorptionsartikel nach Anspruch 5, wobei das erste Delta E* kleiner als das zweite Delta E* Wert gemäß dem hierin definierten Farbmessverfahren ist.

7. Absorptionsartikel nach einem der vorstehenden Ansprüche, wobei Farbstoff auf einer Oberfläche der Oberschicht (20) angeordnet ist.

8. Absorptionsartikel nach einem der vorstehenden Ansprüche, wobei die Oberschicht (20) ein Vliesmaterial umfasst.

9. Absorptionsartikel nach einem der vorstehenden Ansprüche, wobei die Unterschicht (50) einen Farbstoff umfasst, und wobei der Farbstoff auf gegenüberliegenden Seiten der Absorptionskernzone (140) angeordnet ist.

10. Absorptionsartikel nach Anspruch 9, wobei ein erster Farbstoffabschnitt (200) auf einer ersten Seite der Absorptionskernzone (140) angeordnet ist und ein zweiter Farbstoffabschnitt (250) auf einer zweiten Seite der Absorptionskernzone (140) angeordnet ist.

11. Absorptionsartikel nach einem der vorstehenden Ansprüche, ferner umfassend eine optionale Schicht (30), die zwischen der Oberschicht (20) und dem Absorptionskern (40) oder zwischen dem Absorptionskern (40) und der Unterschicht (50) angeordnet ist, wobei die optionale Schicht (30) in der Absorptionskernzone (140) und in der inneren Grenzzone (160), aber nicht in der äußeren Grenzzone (150) angeordnet ist.

12. Absorptionsartikel nach Anspruch 11, wobei die optionale Schicht (30) eine Aufnahme-/Verteilungsschicht oder eine Aufnahmeschicht und eine separate Verteilungsschicht umfasst.

13. Absorptionsartikel nach Anspruch 11, wobei die optionale Schicht (30) eine Trägerschicht umfasst, die in dem hinteren Abschnitt (110) und mittleren Abschnitt (105), aber nicht in dem vorderen Abschnitt (100) angeordnet ist.

**Revendications**

1. Article absorbant jetable (101) ayant un axe longitudinal et un axe transversal disposé perpendiculairement à l'axe longitudinal, une partie avant (100), une partie arrière (110), et une partie centrale (105) disposée entre la partie avant (100) et la partie arrière (110), l'article absorbant jetable (10) comprenant en outre :

une feuille de dessus (20) ;
une feuille de fond (50) ;
un système absorbant disposé entre la feuille de dessus (20) et la feuille de fond (50), dans lequel le système absorbant comprend une âme absorbante (40) ayant des bords latéraux longitudinaux (40A, 40B) sur des côtés opposés de l'âme absorbante (40) et des bords d'extrémité sur des côtés opposés de l'âme absorbante (40), l'âme absorbante (40) étant disposée dans une zone d'âme absorbante (140), dans lequel la zone d'âme absorbante (140) présente une première valeur L* ;

une périphérie externe (95) formée au moins en partie par une partie de la feuille de dessus (20) et une partie de la feuille de fond (50) ;

une zone de limite externe (150) comprenant la périphérie externe (95), la zone de limite externe (150) ayant une largeur inférieure à 30 mm, dans lequel la zone de limite externe (150) présente une deuxième valeur L* inférieure à 89 selon la méthode de mesure de couleur définie ici ;

une zone limite interne (160) disposée entre la zone limite externe et la zone d'âme absorbante (140) dans la partie arrière (110), la zone limite interne (160) présente une troisième valeur L* selon la méthode de mesure de couleur définie ici qui est supérieure à la deuxième valeur L* ; et

dans lequel la première valeur L* est supérieure aux deuxième et troisième valeurs L* de sorte qu'il existe une différence visuelle perceptible entre la zone d'âme absorbante (140) et la zone limite interne (160) ;

dans lequel la feuille de dessus (20) a une opacité allant de 23 à 51 mesurée selon la méthode de mesure d'opacité définie ici ; et

dans lequel le colorant est disposé sur une surface de la feuille de fond (50) ; dans lequel les valeurs L* sont mesurées selon la méthode de mesure de couleur définie ici.

2. Article absorbant selon la revendication 1, dans lequel la deuxième valeur L* est comprise entre environ 50 et 89 selon la méthode de mesure de couleur définie ici.

3. Article absorbant selon l'une ou l'autre des revendications précédentes, dans lequel la troisième valeur L* est comprise entre environ 60 et 94 selon la méthode de mesure de couleur définie ici.

4. Article absorbant selon l'une quelconque des revendications précédentes, dans lequel une première valeur delta E* entre la zone d'âme absorbante (140) et la zone limite interne (160) est supérieure à 4, plus préférablement supérieure à 7, ou le plus préférablement supérieure à 9 selon la méthode de mesure de couleur définie ici.

5. Article absorbant selon l'une quelconque des revendications précédentes, dans lequel une seconde valeur delta E* entre la zone limite interne (160) et la zone limite externe (150) est supérieure à 4, plus préférablement supérieure à 6, ou le plus préférablement supérieure à 8 selon la méthode de mesure de couleur définie ici.

6. Article absorbant selon la revendication 5, dans lequel la première valeur delta E* est inférieure à la seconde valeur delta E* selon la méthode de mesure de couleur définie ici.

7. Article absorbant selon l'une quelconque des revendications précédentes, dans lequel le colorant est disposé sur une surface de la feuille de dessus (20).

8. Article absorbant selon l'une quelconque des revendications précédentes, dans lequel la feuille de dessus (20) comprend un matériau non tissé.

9. Article absorbant selon l'une quelconque des revendications précédentes, dans lequel la feuille de fond (50) comprend un colorant, et dans lequel le colorant est disposé sur des côtés opposés de la zone d'âme absorbante (140).

10. Article absorbant selon la revendication 9, dans lequel une première partie de colorant (200) est disposée sur un premier côté de la zone d'âme absorbante (140), et une seconde partie de colorant (250) est disposée sur un second côté de la zone d'âme absorbante (140).

11. Article absorbant selon l'une quelconque des revendications précédentes, comprenant en outre une couche facultative (30) disposée entre la feuille de dessus (20) et l'âme absorbante (40) ou entre l'âme absorbante (40) et la feuille de fond (50), dans lequel la couche facultative (30) est disposée dans la zone d'âme absorbante (140) et dans la zone limite interne (160), mais pas dans la zone limite externe (150).

12. Article absorbant selon la revendication 11, dans lequel la couche facultative (30) comprend une couche d'acquisition/distribution ou une couche d'acquisition et une couche de distribution séparée.

13. Article absorbant selon la revendication 11, dans lequel la couche facultative (30) comprend une couche de support qui est disposée dans la partie arrière (110) et la partie centrale (105), mais pas dans la partie avant (100).

FIG. 1A

EP 4 304 541 B1

EP 4 304 541 B1

FIG. 1B

FIG. 1C

FIG. 2

FIG. 3

315

305

40A

EP 4 304 541 B1

REFERENCES CITED IN THE DESCRIPTION

**Patent documents cited in the description**

- US 2012310200 A1
- WO 20192333569 A1
- US 8715258 B2, Munakata
- US 3929135 A, Thompson
- US 4324246 A, Mullane
- US 4342314 A, Radel
- US 4463045 A, Ahr
- US 5006394 A, Baird
- US 4609518 A, Curro
- US 4629643 A, Curro
- US 9700463 B
- US 9849602 B
- US 5885265 A, Osborn, III
- US 6462251 B, Cimini
- US 6623464 B, Bewick-Sonntag
- US 6664439 B, Arndt
- US 3881489 A
- US 4341216 A
- US 4713068 A
- US 4818600 A
- EP 203821 A
- EP 710471 A
- EP 710472 A
- EP 793952 A
- GB 2184389 A
- GB 2184390 A
- GB 2184391 A
- US 4591523 A
- US 3989867 A
- US 3156242 A
- WO 9724097 A
- US 6436508 B, Ciammaichella
- US 5599335 A, Goldman
- EP 1447066 A, Busam
- WO 9511652 A, Tanzer
- US 20080312622 A1, Hundorf
- WO 2012052172 A
- US 4610678 A, Weisman
- US 4673402 A, Weisman
- US 4888231 A, Angstadt
- US 4834735 A, Alemany
- US 5234423 A, Alemany
- US 5147345 A
- US 20180098893
- US 20180098891 A
- US 20150351976 A1
- US 20140343523 A1
- US 729704